# EUROPÄISCHE PATENTANMELDUNG

(11) **EP 4 745 226 A1**
(43) Veröffentlichungstag der Anmeldung: **20.05.2026**
(21) Anmeldenummer: 24214057.2
(22) Anmeldetag: 19.11.2024
(51) Int. Cl.: C12N 5/00, A23K 10/30, A23K 50/80, A23K 10/22, A23L 17/20

(54) **VERFAHREN ZUR HERSTELLUNG VON FISCHZELLEN**

(71) Anmelder: Kaesler Nutrition GmbH, 27472 Cuxhaven (DE)
(72) Erfinder: SCHMIDT, Mario, 31655 Stadthagen (DE); KRUSE, Georg, 27570 Bremerhaven (DE)
(74) Vertreter: Simandi, Claus

(57) **Zusammenfassung**

Gegenstand der vorliegenden Erfindung ist ein Verfahren zur Herstellung von Fischzellen mittels eines Zellmediums, und daraus erhältliche Fischprodukte und deren Verwendung.

## Beschreibung

Gegenstand der vorliegenden Erfindung ist ein Verfahren zur Herstellung von Fischzellen mittels eines Zellmediums, und daraus erhältliche Fischprodukte und deren Verwendung.

Fleisch ist eine wichtige Proteinquelle in der menschlichen Ernährung. Der umstrittene Tierschutz in der traditionellen Fleischindustrie sowie die wachsende Weltbevölkerung und die steigende Nachfrage nach Fleischprodukten, insbesondere Fisch(fleisch)produkte machen nachhaltige Produktionsalternativen unverzichtbar. Die Technologie des kultivierten Fleisches (Cultivated Meat, insbesondere so genannter Clean Fish) bietet die Möglichkeit, essbare tierische Proteinquellen zu produzieren, die nicht mit den Umweltauswirkungen der Tierhaltung verbunden sind.

Der Herstellungsprozess von Cultured Meat kann wie folgt beschrieben werden:
Alle in Fleisch enthaltenen Zell-Substanzen (Muskel, Bindegewebe, Fett) können in jeweiligen Bioreaktoren auf Basis von Zellkulturen hergestellt werden, es folgt ein mehrstufiger Prozess (McClements, D. J. (2023) Biotech Meat: Growing Meat from Cells. Meat Less - The Next Food Revolution, S. 149 ff. Springer-Verlag GmbH).

Phase 1, Sammelphase: Zellentnahme zumeist mittels Muskelbiopsie, es folgt eine Zellauswahl und anschließende Herstellung einer Spenderzellbank mittels Zellkultur. In dieser Zellkultur können die ausgewählten Stammzellen den Prozess der Immortalisierung unterlaufen, so dass sie unbegrenzt zur Produktion verwertet werden können.

Phase 2, Proliferationsphase: Zellen der Spenderzellbank werden unter kontrollierten Bedingungen angezüchtet, hierbei kommt eine Nährlösung mit Nährstoffen und Wachstumsfaktoren zum Einsatz. Die Anzucht wird im Bioreaktor unter kontrollierten und überwachten Bedingungen fortgesetzt, insbesondere Temperatur, Sauerstoff-Gehalt, pH-Wert, so dass die Zellen proliferieren.

Phase 3, Reifungsphase: Überführung der Zellen in weitere Bioreaktoren unter jeweiliger Zugabe spezifischer Additive wie Nährstoffe und Wachstumsfaktoren. So entstehen je nach gewählten Additiven unterschiedliche Gewebe: Muskelzellen, Bindegewebezellen oder Fettzellen. Unter Zugabe von Trägermaterialien ("scaffolds") können bereits im Reaktor Zellverbände entstehen, die den natürlichen Zellverbänden in echtem Fleisch ähneln.

Nach 4-6 Wochen Reifezeit ist der Prozess abgeschlossen und aus den entstandenen Zellen oder Zellverbänden kann das Fleischprodukt hergestellt werden.

Weiterhin ist im Stand der Technik bevorzugt, dass Serum-freie Zellmedien, also ohne Fötalem Kälberserum (FCS), verwendet werden, insbesondere zur Vermeidung von Kontaminationsrisiken, und bessere Reproduzierbarkeit (siehe z.B. EP4251740A1).

Im Rahmen dieser Erfindung ist Fischfleisch bevorzugt.

Der Begriff "Fischfleisch" oder "Fischfilet" bezeichnet das essbare Muskelgewebe ohne Haut, Gräten und Innereien von Fischen.

Je nach natürlicher Fischart unterscheidet sich Fischfleisch in Geschmack, Konsistenz und Nährwert. Es gibt fettärmere Sorten wie Kabeljau oder Hecht sowie fettreichere als Lachs und Makrele, die höhere Mengen an Omega-3-Fettsäuren enthalten.

Es besteht das Bedürfnis, kultiviertes Fischfleisch dem nativen Fischfleisch in Form, Textur, Farbe, Geschmack und Qualität nachzukommen.

Im Stand der Technik ist die Herstellung von in-vitro Fischzellen beschrieben. EP 2 500 412 B1 beschreibt die Produktion von Fischzellen, wobei die Kultivierung bei zwei verschiedenen Temperaturen erfolgt, und das Zellkulturmedium einen Zusatz von mindestens einer kurzkettigen, mehrfach ungesättigten Fettsäure, die aus der Gruppe aus Alpha-Linolensäure (ALA, C18:3n3), Palmitoleinsäure (C16:1n7), Ölsäure (C18:1n9) und Linolsäure (C18:2n6) ausgewählt ist, enthält.

Im Stand der Technik ist jedoch nachteilig, dass Fischzellen nicht in ausreichender Ausbeute und Qualität kostengünstig hergestellt werden können.

Die Erfinder haben festgestellt, dass das Aminosäureprofil für die Fischzellenvermehrung bzw. Proliferation wesentlich ist, und qualitativ zu verbesserten Fischzellen führt, ohne dass z.B. spezielle Fettsäuren eingesetzt werden müssen.

Überaschenderweise können Hydrolysate aus Gluten *und* Hefe vorteilhafte Aminosäureprofile für ein Zellmedium bereitstellen, die die Fischzellenvermehrung fördern, insbesondere bei anspruchsvollen Fischzellen, wie Lachs. Insbesondere ist vorteilhaft, dass der Einsatz eines Serums (wie FCS) nicht vonnöten ist.

Weiterhin wird aufgrund des erfindungsgemäßen Zellmediums überaschenderweise ein besonders geeignetes und vorteilhaftes Fettsäurespektrum für die Zellkultur sofort erreicht, so dass die kultivierten Fischzellen ein vergleichbares Lipidspektrum aufweisen, wie z.B. von Garnelen (siehe Abbildungen).

Gegenstand der Erfindung ist daher ein Verfahren zur Herstellung von Fischzellen, wobei folgende Schritte durchgeführt werden:
a) Vorlegen von mindestens einer proliferationsfähigen Fischzelle,
b) Bereitstellen von Salzen, Vitaminen und Spurenelementen,
c) Bereitstellen eines Hydrolysats aus Gluten und Hefe,
d.) Versetzen von c.) mit b.) unter Erhalten eines Zellmediums und Durchführen einer Zellkultivierung mit a.).

Das erfindungsgemäße Verfahren erlaubt die vorteilhafte Herstellung bzw. Produktion, Anreicherung und Vermehrung von Fischzellen aus mindestens einer proliferationsfähigen Fischzelle.

In einer bevorzugten Ausführungsform der Erfindung erfolgt das Bereitstellen des Hydrolysats von c.) durch Verwendung von mindestens einem Pflanzenöl, vorzugsweise solche Pflanzenöle, die gesättigte und ungesättigte Fettsäuren enthalten.

Die Hydrolyse zur Herstellung des Hydrolysats von c.) kann chemisch oder enzymatisch erfolgen. Die enzymatische Hydrolyse mithilfe einer Protease ist bevorzugt.

Daher betrifft die Erfindung ein Hydrolysat von c.), die eine Emulsion ist, und ein Pflanzenöl enthält, folglich ein Bereitstellen eines Hydrolysats aus Gluten und Hefe, und mindestens einem Pflanzenöl, wobei das Hydrolysat mit einem Pflanzenöl versetzt ist oder dieses enthält.

In einer weiteren besonders bevorzugten Ausführungsform ist das mindestens eine Pflanzenöl, ausgewählt aus der Gruppe Weizenkeimöl (174-176 mg/100 g), Sonnenblumenöl (60 mg/100 g), Leinsamenöl (57 mg/100 g), Walnussöl (39 mg/100 g), Maiskeimöl (31-34 mg/100 g), Distelöl (29-45 mg/100 g), Sesamöl (28 mg/100 g), Erdnussöl (25 mg/100 g), Rapskernöl (25 mg/100 g), Mandelöl(25 mg/100 g), Palmöl (25 mg/100 g), Rapsöl (20 mg/100 g), Sojaöl (17-25 mg/100 g), Olivenöl (12-40 mg/100 g), Schwarzwurzelöl (6 mg/100 g) oder Leinöl (5,8 mg/100 g).

Die Klammern weisen den Anteil an Vitamin E im jeweiligen Öl aus.

In einer weiteren Ausführungsform können neben dem Pflanzenöl, weitere Hilfs- und Zusatzstoffe enthalten sein, wie Antioxidationsmittel, pH-Stabilisatoren, etc., insbesondere solche wie Tocopherol (Vitamin E), Polyphenole, Zitronensäure u.a.

Eine entsprechende Zusammensetzung für die Bereitstellung des Hydrolysats enthält 15- 35 Gew.%, insbesondere 23,8% Gew. % Weizengluten, 15- 35 Gew.%, insbesondere 23,8% Gew. % Hefeextrakt, 40- 60 Gew.%, insbesondere 47,6 Gew.% Pflanzenöl, ggfs. weitere Hilfs- und Zusatzstoffe, wie optional 1 - 5 Gew.%, insbesondere 2,4 Gew.% Zitronensäure und optional 1 - 5 Gew.%, insbesondere 2,4% Gew.% Tocopherol (Vitamin E).

In einer bevorzugten Ausführungsform wird eine solche Zusammensetzung erhitzt, da vor allem Mehlfraktionen wie das Gluten bakterielle und/oder fungale Sporen beinhalten können, die inaktiviert werden sollen, um im späteren Zellkulturprozess steril vorzuliegen. Das Antioxidans Tocopherol kann zu der Zusammensetzung gegeben werden, um die Lipide bei hohen Temperaturen vor Oxidation zu schützen.

Das Erhitzen dieser Zusammensetzung erfolgt vorzugsweise bei 100 - 180 °C insbesondere bei 130 - 150 °C für 10 - 180 Minuten, insbesondere 30 - 60 Minuten. Die erhaltene Emulsion wird anschließend einer enzymatischen oder chemischen Hydrolyse unterzogen. Die enzymatische Hydrolyse erfolgt mit einer Protease (Peptidase, Proteinase oder proteolytisches Enzyme) unter Freisetzung der Aminosäuren.

Nach Beendigung der Hydrolyse können feste Bestandteile durch Pelletierung in der Zentrifuge oder Filtrierung entfernt und das ölhaltige Gemisch mithilfe von Ultraschall emulgiert werden.

Daher betrifft die Erfindung die Herstellung des Hydrolysats gemäß c.), wobei Gluten und Hefe mit einem Pflanzenöl versetzt und erhitzt werden, und anschließend chemisch oder enzymatisch hydrolysiert werden.

Anschließend erfolgt das Versetzen des Basismediums oder Basalmediums aus a.) mit dem Hydrolysat (supra). Die Osmolarität des erhaltenen Zellmediums wird mit sterilem VE-Wasser und einer 30%-igen NaCl auf vorzugsweise 290-320 mOsmol/kg eingestellt.

Das Medium ist vorteilhaft lagerstabil und zeigt auch nach längerer Zeit (6 Wochen) keine Präzipitatbildung, welche oftmals ein Problem bei der Zellkulturmedienherstellung darstellt. Es ist zudem bevorzugt, Calciumlactat im erfindungsgemäßen Zellmedium einzusetzen, da Calciumlactat die vorteilhafte Stabilisierung des Zellmediums besonders unterstützt.

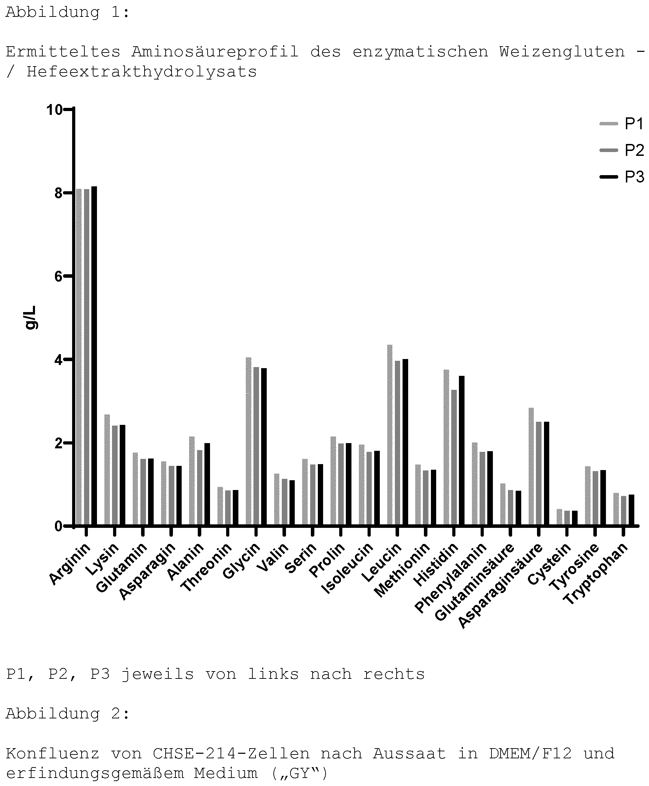

Insbesondere das erfindungsgemäße Hydrolysat nimmt als Aminosäurelieferant eine essenzielle Rolle im Zellmedium ein. Das Aminosäureprofil des Weizengluten/Hefeextrakt-Hydrolysats wurde analysiert (Abb. 1). Hierbei stellt sich heraus, dass Hefe und Gluten das Aminosäurenprofil komplettieren, um den Fischzellen die benötigten Aminosäuren optimal zur Verfügung zu stellen.

Weiterhin kann das erfindungsgemäße Zellmedium eine beliebige Kohlenstoffquelle enthalten, wie Zucker, insbesondere Glucose.

Die erfindungsgemäß zur Zellkultivierung vorgesehenen proliferationsfähigen Fischzellen können beliebig sein. Es können sowohl bekannte immortalisierte Fischzelllinien des Standes der Technik verwendet als auch neue Zelllinien aus Fischgewebe eingesetzt werden. Das Ausgangsgewebe für eine Fischzell-Primärkultur kann z.B. das Gesamtgewebe einer Fischlarve oder ein bestimmtes Organ eines adulten Zielfisches sein. Als adultes Fischgewebe kann beispielsweise Gewebe aus einer Niere, insbesondere der Kopfniere, der Leber, dem Pankreas bzw. den Pylorusanhängen, Darm, Herz, Gehirn, den Gonaden, dem Fettgewebe, der Haut, oder Muskelgewebe verwendet werden.

In einer bevorzugten Ausführungsform wird das Gesamtgewebe einer Fischlarve verwendet. Der Begriff "Fischlarve" umfasst insbesondere Fischeier.

Erfindungsgemäß verwendete proliferationsfähige Fischzellen sind weiterhin nicht abschließend Fischlarven oder Fischgewebe aus beliebigen Fischspezies, vorzugsweise jedoch solche der Osteichthyes, insbesondere Spezies der Teleostei. Beispiele hierfür sind Spezies der Heringsfische (Clupeoidei), Lachsartigen (Salmonoidei), z.B. Lachse, Forellen, insbesondere Bachforelle, Regenbogenforelle, Saiblinge, Huchen, Felchen, Renken, Äschen, Stinte, der Karpfenfische (Cyprinidae), Aale (Anguillidae), Barsche (Percidae), Dorsche (Gadidae), Welse (Siluroidae), Plattfische (Pleuronectiformes), Hornhechte (Beloniformes),Störe (Acipenseriformes) etc. Bevorzugt sind jedoch Stör, Hering, Forelle, Lachs, Aal, Karpfen, Makrele, Heilbutt, Sardine.

Die proliferationsfähigen Fischzellen können als Suspensionszellen vorliegen oder ein Wachstum kann auf (Micro-)Carriern (z.B. Pflanzenpartikel u.a. Träger) erfolgen. Die Aussaat kann in einem Bioreaktor (ggfs. kontinuierliche Perfusionskultur notwendig) mit dem erfindungsgemäßen Zellmedium erfolgen, bzw. die proliferationsfähigen Fischzellen werden in Kontakt mit dem Zellmedium gebracht.

In einer weiteren Ausführungsform betrifft die Erfindung daher ein erfindungsgemäßes Verfahren, wobei mindestens ein Bioreaktor eingesetzt wird.

"Gluten" ist ein wertvolles Protein-Gemisch, das hauptsächlich in den Getreidesorten Weizen, Roggen, Gerste und verwandten Getreidearten wie Dinkel vorkommt, und Gliadine und Glutenine enthält. Weizengluten ist bevorzugt.

"Hefe" oder "Hefeextrakt" ist ein wertvoller Nährstoff, reich an B-Vitaminen, Mineralstoffen und wertvollen Proteinen.

Die Bioreaktoren können mit Rührer und anderen üblichen Hilfsmitteln ausgestattet sein. Geeignete Bioreaktoren zur Kultivierung sind solche wie Hohlfaser-Bioreaktoren, Rührkesselreaktoren (Fermenter), Wirbelschichtreaktoren (Aggregate oder poröse Trägermaterialien), Festbettreaktoren mit Hohlfaser- bzw. Flachbettmembranen (Verfahren ist Tangentialflussfiltration), Plug-Flow-Reactor, Spinner Flaschen Kultivierung (siehe Horst Chmiel (Hrsg.) Bioprozesstechnik (2011), Spektrum Akademischer Verlag, Heidelberg). Die Bioreaktoren können das erfindungsgemäße Medium, Kulturmedium oder Kulturflüssigkeit enthalten.

Weiterhin betrifft die Erfindung ein Verfahren zur Gewinnung von Fischprodukten, ausgewählt aus der Gruppe Fischmehl, Fischproteinen, Fischölen und Fettsäuren, Fischfett, Fischfleisch, aus Fischzellkulturen umfassend:
i.) Kultivieren und Vermehren von proliferierenden Fischzellen in einem Zellkulturmedium gemäß dem erfindungsgemäßen Verfahren und Erhalten von Fischzellen,
ii.) Entnahme der (erhaltenen) Fischzellen,
iii.) entweder Gefriertrocknen und/oder Zerkleinern und/oder Aufschließen der entnommenen Fischzellen,
iv.) oder Bereitstellen der entnommenen in einen Verbund von Fischzellen für Fischfett oder Fischfleisch oder Fischprodukte davon, insbesondere Fischfilet, Pets für Fischburger, Backfisch, Fischstäbchen.

"Entnehmen" im Sinne dieser Erfindung bedeutet isolieren oder ernten der kultivierten Fischzellen.

Ein Verbund von Fischzellen kann beispielsweise mithilfe von Pressen oder mit einem 3D-Drucker hergestellt werden.

Fischprodukte aus einem solchen Verbund von Fischzellen sind insbesondere bearbeitete Produkte, wie Fischfilet, Pets für Fischburger, Backfisch oder Fischstäbchen, ggfs. mit Zusätzen von weiteren Zusatz- und Hilfsstoffen.

Es sei darauf hingewiesen, dass Merkmale, die im Zusammenhang mit einer beispielhaften Ausführungsform oder einem beispielhaften Gegenstand beschrieben werden, mit jeder anderen beispielhaften Ausführungsform oder mit jedem anderen beispielhaften Gegenstand kombiniert werden können.

Wenn ein Begriff mit einem unbestimmten oder bestimmten Artikel, wie zum Beispiel "ein" im Singular bezeichnet wird, schließt dies auch den Begriff im Plural mit ein und umgekehrt, sofern der Kontext nicht eindeutig anderes festlegt.

Der Ausdruck "umfassen", wie er hier verwendet wird, schließt nicht nur die Bedeutung von "enthalten" ein, sondern kann auch "bestehen(d) aus" und "im Wesentlichen bestehend aus" bedeuten.

Zellmedium oder Zellkulturmedium sind synonym zu lesen.

Nachfolgende Beispiele und Abbildungen dienen zur näheren Erläuterung der Erfindung, ohne jedoch die Erfindung auf diese Beispiele und Abbildungen zu begrenzen.

Beispiele und Abbildungen:

### Beispiel 1:

### Herstellung des erfindungsgemäßen Zellmediums

Um das erfindungsgemäße Zell(kultur)medium ("GY") herzustellen sind insgesamt zwei Teilschritte nötig, um ein Komplettmedium zu generieren. Die erste Komponente ist ein Gemisch aus anorganischen Salzen, Vitaminen und Spurenelementen, die in üblicher Weise sterilfiltriert werden ("Basismedium" - 33 Komponenten, siehe Beispiel unten).

Die zweite Komponente besteht aus Hefeextrakt und Weizengluten, die als Aminosäurequelle dienen und zudem ein Pflanzenöl, vorzugsweise Rapsöl, das ebenfalls hinzugegeben wird. Hierbei ist zu beachten, dass Rapsöl zum einen als Wärmeüberträger in einem vorgeschalteten Sterilisierprozess dient als auch als die Fettsäurequelle für die Zellkultur darstellt. Ein nächster Schritt ist das Erhitzen dieser Komponenten (Zusammensetzung), da vor allem Mehlfraktionen wie das Gluten bakterielle und/oder fungale Sporen beinhalten können, die inaktiviert werden sollen, um im späteren Zellkulturprozess steril vorzuliegen. Diese Komponenten werden anteilig (Massenprozent, Gew. %) mit 23,8% Weizengluten, 23,8% Hefeextrakt, 47,6% Rapsöl, 2,4% Zitronensäure und 2,4% Tocopherol (Vitamin E) verwendet. Das Antioxidans Tocopherol wird zu dem Gemisch gegeben, um die Lipide bei hohen Temperaturen vor Oxidation zu schützen. Ebenfalls wird Vitamin E hinzugegeben, um auch den physiologischen Bedarf der Fischzellen decken zu können. Das Erhitzen dieser Komponenten findet bei 130°C für 30 Minuten statt. Eine Sterilisierung und Funktionalität dieser Proben wurde durch Abklatschplatten gezeigt. Die entstandene Emulsion wird anschließend direkt für den enzymatischen Hydrolyseschritt eingesetzt. Hierbei werden 10% (m%) des Hydrolysats in einem 50 mM Essigsäureacetatpuffer pH 5,0 eingesetzt, in den eine Protease von Aspergillus oryzae mit mindestens 4000 Units/L zugesetzt wird. Unter sterilen Bedingungen wird dann anschließend für 16 Stunden bei 50°C inkubiert, um Aminosäuren freizusetzen. Danach wird das Enzym bei 95°C für 10 Minuten wieder inaktiviert. Feste Bestandteile werden durch Pelletierung in der Zentrifuge entfernt und das Öl/Wasser-Gemisch nochmals im Ultraschallbad emulgiert. Anschließend erfolgt das Zusammenführen des Basismediums mit dem Hydrolysat, wobei zwischen 1 und 10 % (Vol.-%) eingesetzt werden, die Osmolarität des Komplettmediums (GY-Medium) wird mit sterilem VE-Wasser und einer 30%i-gen NaCl auf 290-320 mOsmol/kg eingestellt.

### Beispiel 2:

### Lachszellen mit Fettsäureprofil ähnlich zu Garnelenfleisch

Da zu diesem Zeitpunkt noch keine Adaptation mit Medium komplett ohne Serum durchgeführt wurde, wurde zumindest eine Serumreduktion auf 2% FBS erreicht. Die Zellen wurden dabei im entwickelten Medium kultiviert, bis eine ungefähre Feuchtmasse von 150 mg pro zu analysierende Probe erreicht wurde. Zum Vergleich wurden bis auf die Regenbogenforelle (stammt aus der Aquakultur des Thünen-Instituts) Fische (Fischfleisch) und Garnelen aus freier Wildbahn erworben und anschließend ebenfalls mit der Zellkultur analysiert.

### Durch die Verwendung des Systems von

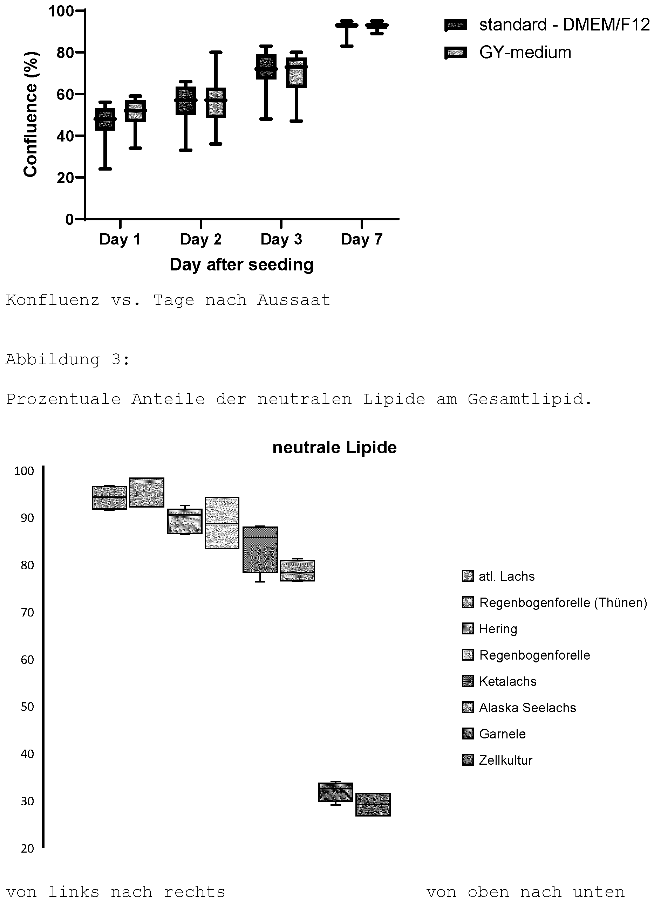

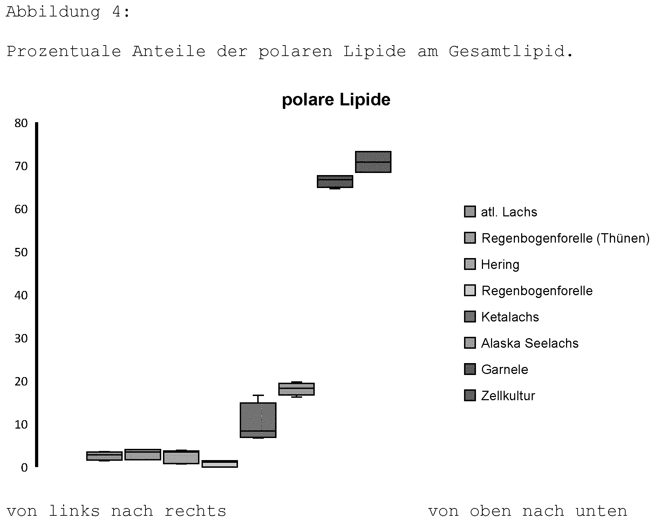

Dünnschichtchromatographie (TLC) mit anschließender Flammenionisationsdetektion (FID) wurden bis zu neun Lipidklassen, welche in Fischlipid zu erwarten sind, zeitgleich identifiziert und quantifiziert. Lösungen der Fischlipide werden dazu auf Silica-beschichtete Glasstäbchen (Chromarods) aufgetragen und die verschiedenen Lipidklassen mittels Dünnschichtchromatographie voneinander getrennt. Dazu sind drei aufeinander folgende Entwicklungsschritte nötig, welche mit verschieden polaren Laufmittelgemischen durchgeführt werden. Im Anschluss an jeden Entwicklungsschritt erfolgt jeweils eine partielle Detektion der Chromarods im FID zur Analyse der, für den entsprechenden Entwicklungsschritt relevanten Lipidklassen. Über Integrale der Signale können die Mengen (in pg) der jeweiligen Lipidklasse bestimmt werden, woraus sich eine prozentuale Verteilung ergibt. Wie in den Abbildungen zu sehen ist, ähneln sich die Proben in der Zusammensetzung von neutralen (Abb. 3) und polaren (Abb. 4) Lipiden sehr. Somit wurde gezeigt, dass vor allem polare Lipide, die eine hohe Bedeutung in der Ernährung spielen vor allem in den kultivierten Zellen vorkommen.

### Beispiel 3:

Ausführungsform eines erfindungsgemäßen Zellmediums ("GY")

| **Basismedium** | **[g/L]** |
|---|---|
| NaCl | 6,00000 |
| KCI | 0,31199 |
| Ca-Lactat | 0,10911 |
| MgSO₄ * 7H₂O | 0,14343 |
| Na₂HPO₄ * 2H₂O | 0,15850 |
| NaHCO₃ | 2,43805 |
| Phenolrot | 0,00400 |
| Glucose | 2,00000 |

| **Vitamin-Lösung** | **[mg/L]** |
|---|---|
| Ascorbinsäure | 0,7600 |
| Biotin | 0,0037 |
| Folsäure | 0,6621 |
| Nicotinamid | 2,0186 |
| Calcium-D(+)-pantothenat | 2,2392 |
| Vitamin B6 (Pyridoxine) | 1,6707 |
| Riboflavin | 0,2183 |
| Thiamine | 1,7062 |
| Vitamin B12 | 0,6777 |
| Cholinchlorid | 8,9810 |
| Inositol (Meso-Inosit) | 12,6085 |
| Ethanolamin | 0,9467 |
| Putrescin | 0,1322 |

| **Spurenelemente** | **[mg/L]** |
|---|---|
| CuSO₄ | 0,00142 |
| FeSO₄ | 0,22786 |
| ZnSO₄ | 0,24221 |
| NH₄VO₃ | 0,00015 |
| Na₂SeO₃ | 0,00251 |

| **Hydrolysat** | **[g/L]** |
|---|---|
| enzymatisches Hydrolysat | 1 -10 |
| aus Weizengluten und | |
| Hefeextrakt und | |
| Pflanzenöl | |

### Beispiel 4:

Die Fettsäureprofile aller Zellproben sind untereinander ähnlich, wie in Tabelle 1 zu sehen ist. Alle werden durch Ölsäure (C18:1n9) dominiert, welche zwischen 47 und 57 % der gesamten Fettsäuren ausmacht, gefolgt von Palmitinsäure (C16:0) und Stearinsäure (C18:0). Derartige Ölsäure-Anteile treten unter den Vergleichsproben nur bei den fettreichen Zuchtfischen (Forelle; atl. Lachs) auf. Andere Fettsäuren treten maximal in Anteilen von 5 % auf. Mehrfach ungesättigte Fettsäuren konnten, wenn auch in deutlich niedrigeren Anteilen als in den Fischvorbildern nachgewiesen werden. Hier unterscheiden sich zwischen den Zellen die Fettsäurederivate und Anteile allerdings voneinander. Während bei Königslachs (mit 2 % FBS kultiviert) vor allem die C18-PUFA Vertreter nachgewiesen werden konnten, entfallen bei den anderen Zellproben Anteile bis zu 4 % auf die LC-PUFA ARA, EPA oder DHA. Ihre Anteile liegen jedoch deutlich niedriger als bei den Fischproben, welche einen vergleichbaren Gesamtlipidgehalt aufweisen (vgl. Abb.3). Vergleichbare Anteile LC-PUFA finden sich nur bei fettreichen Fischen, wobei hier zum einen die Gehalte an ARA deutlich geringer sind und zum anderen hohe Absolutwerte beispielsweise für EPA und DHA erreicht werden. Die entsprechenden Unterschiede sind in Abbildung 4 deutlich zu erkennen.

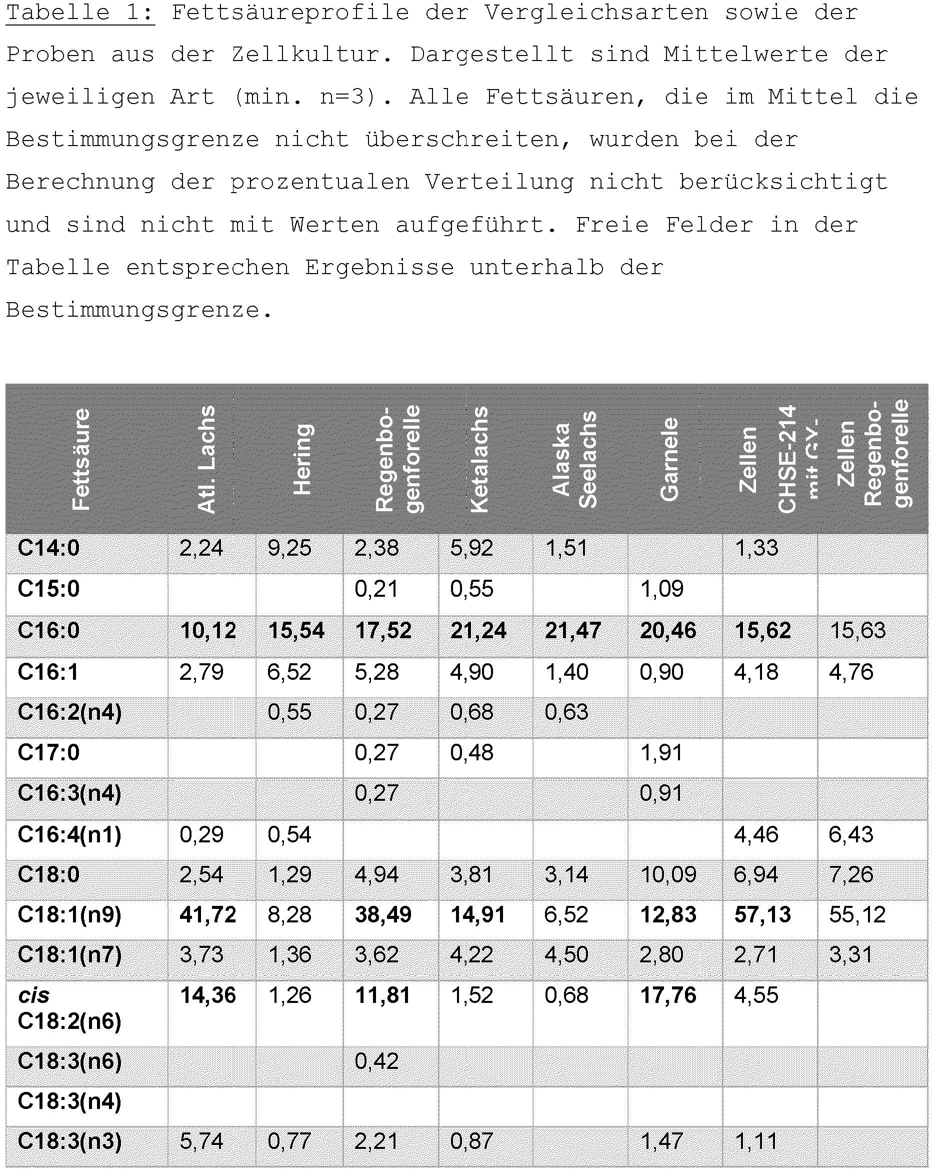

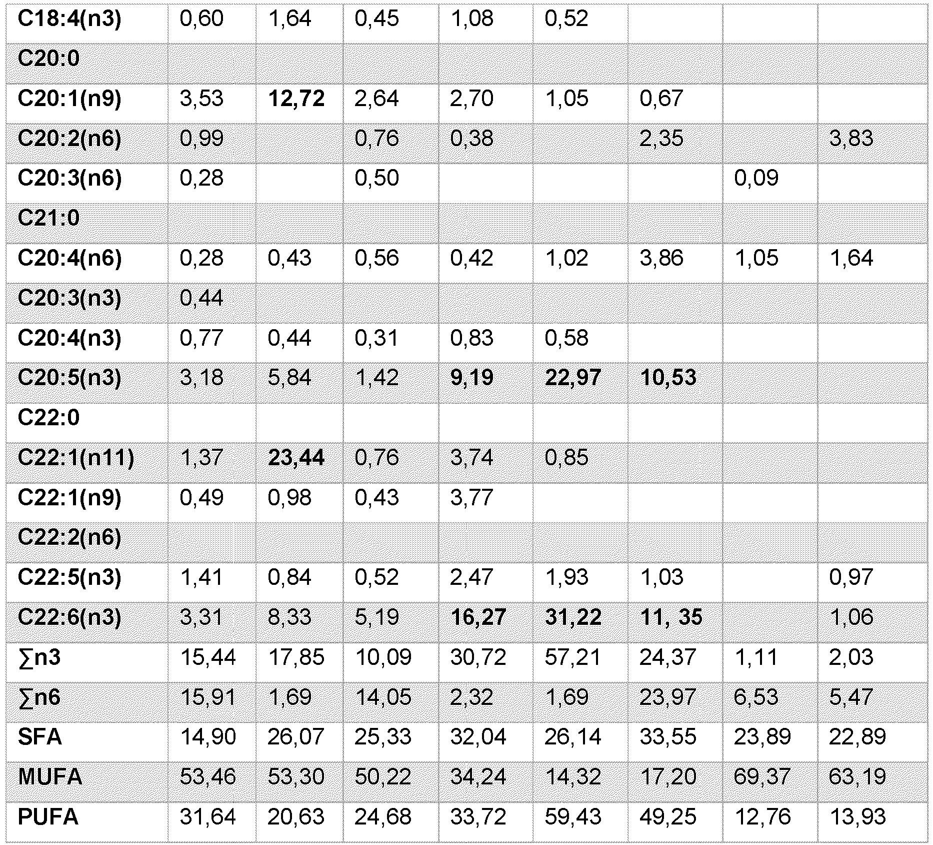

Abbildung 1: Ermitteltes Aminosäureprofil des enzymatischen Weizengluten - / Hefeextrakthydrolysats. Die Gehalte der 20 proteinogenen Aminosäuren in den Hydrolysaten wurden über Standards mittels Ionenchromatographie bestimmt. Gezeigt sind die Gehalte von drei Replikaten (P1 bis P3, n=3).

Abbildung 2: Konfluenz von CHSE-214-Zellen nach Aussaat in DMEM/F12 und erfindungsgemäßem Medium. Die Zellen wurden zuvor in DMEM/F12 kultiviert, trypsiniert und zentrifugiert, die Pellets wurden dann resuspendiert und in das jeweilige Medium ausgesät. Für die Analyse der Konfluenz wurden die Software Zeiss Labscope mit dem AI Cell Confluency Modul verwendet und die Messungen manuell bestätigt.

Abbildung 3: Prozentuale Anteile der neutralen Lipide am Gesamtlipid. Dargestellt sind die Mittelwerte von allen Vergleichsfischen, sowie Garnele und der Zellkultur (CHSE-214 mit erfindungsgemäßem Medium). Von links nach rechts nimmt der Fettgehalt der Vergleichsfischproben ab.

Mittlere Fettgehalte/Probenzahl:
Atlantischer Lachs: Lip% = 12,4 %, n = 4; Regenbogenforelle (Thünen): Lip% = 11,7 %, n = 3; Hering: Lip% = 9,1 %, n = 5; Regenbogenforelle: Lip% = 8,44 %, n = 3; Ketalachs: Lip% = 1,45 %, n = 4; Alaska Seelachs: Lip% = 0,74 %, n = 4; Garnele: Lip% = 1,0 %, n = 4; Zellkultur (2 %/10 % FKS): Lip% = 0,6 %, n = 2.

Abbildung 4: Prozentuale Anteile der polaren Lipide am Gesamtlipid. Dargestellt sind die Mittelwerte aller Vergleichsfische, sowie Garnele und der Zellkultur. Von links nach rechts nimmt der Fettgehalt der Vergleichsfischproben ab. Mit Zellkultur ist die CHSE-214-Zelllinie gemeint, die mit dem erfindungsgemäßen Zellmedium kultiviert wurde.

## Patentansprüche

1. Verfahren zur Herstellung von Fischzellen, **dadurch gekennzeichnet, dass** folgende Schritte durchgeführt werden:
a) Vorlegen von mindestens einer proliferationsfähigen Fischzelle,
b) Bereitstellen von Salzen, Vitaminen und Spurenelementen,
c) Bereitstellen eines Hydrolysats aus Gluten und Hefe,
d.) Versetzen von c.) mit b.) unter Erhalten eines Zellmediums und Durchführen einer Zellkultivierung mit a.).

2. Verfahren nach Anspruch 1, **dadurch gekennzeichnet, dass** das Hydrolysat aus c.) mindestens ein Pflanzenöl enthält.

3. Verfahren nach Anspruch 2, wobei zur Herstellung des Hydrolysats gemäß c.) Gluten und Hefe mit einem Pflanzenöl versetzt und erhitzt werden, und anschließend chemisch oder enzymatisch hydrolysiert werden.

4. Verfahren nach Anspruch 3, wobei zur Herstellung des Hydrolysats gemäß c.) Gluten ein Weizengluten ist und Hefe ein Hefeextrakt ist, insbesondere 15- 35 Gew.% Weizengluten und 15- 35 Gew.% Hefeextrakt werden mit 40-60 Gew.% Pflanzenöl versetzt.

5. Verfahren nach Anspruch 3, wobei das Erhitzen bei 100 bis 180 Grad Celsius erfolgt.

6. Verfahren nach einem der vorstehenden Ansprüche, wobei die mindestens eine proliferationsfähige Fischzelle ausgewählt ist, aus der Gruppe Stör, Hering, Forelle, Lachs, Aal, Karpfen, Makrele, Heilbutt oder Sardine.

7. Verfahren nach einem der vorstehenden Ansprüche, wobei die Zellkultivierung in mindestens einem Bioreaktor erfolgt.

8. Verfahren zur Gewinnung von Fischprodukten, ausgewählt aus der Gruppe Fischmehl, Fischproteinen, Fischölen und Fettsäuren, Fischfett, Fischfleisch, aus Fischzellkulturen umfassend:
i.) Kultivieren und Vermehren von proliferierenden Fischzellen in einem Zellkulturmedium gemäß dem Verfahren nach einem der Ansprüche 1 bis 7 und Erhalten von Fischzellen,
ii.) Entnahme der Fischzellen,
iii.) entweder Gefriertrocknen und/oder Zerkleinern und/oder Aufschließen der entnommenen Fischzellen,
iv.) oder Bereitstellen der entnommenen in einen Verbund von Fischzellen für Fischfett oder Fischfleisch oder Fischprodukte davon, insbesondere Fischfilet, Pets für Fischburger, Backfisch, Fischstäbchen.

9. Zellmedium umfassend Salze, Vitamine und Spurenelemente und ein Hydrolysat aus Gluten und Hefe und ein Pflanzenöl, insbesondere Calciumlactat.

10. Zellmedium nach Anspruch 9 gemäß der Ausführungsform nach Beispiel 3.

11. Verwendung eines Zellmediums nach Anspruch 9 oder Anspruch 10 zur Kultivierung von Fischzellen.
